# EUROPÄISCHE PATENTSCHRIFT

(11) **EP 1 114 025 B1**
(45) Veröffentlichungstag und Bekanntmachung des Hinweises auf die Patenterteilung: **29.10.2003**
(21) Anmeldenummer: 99946050.4
(22) Anmeldetag: 27.08.1999
(51) Int. Cl.: C07C 315/02, C07C 317/10, C07C 317/18, C07C 319/20, C07C 323/03, C07C 323/16, C07C 381/12, C07D 333/46

(54) **VERFAHREN ZUR HERSTELLUNG VON DERIVATEN DES 4-ALKYLSULFINYLMETHYLARYLENMETHANOLS**
METHOD FOR PRODUCING DERIVATIVES OF 4-ALKYLSULFINYL METHYLARYLENE METHANOLS
PROCEDE DE PRODUCTION DE DERIVES DU METHANOL 4-ALKYLSULFINYLMETHYLARYLENE

(30) Priorität: 08.09.1998 DE 19840943
(43) Veröffentlichungstag der Anmeldung: 11.07.2001
(73) Patentinhaber: Covion Organic Semiconductors GmbH, 65926 Frankfurt (DE)
(72) Erfinder: VANDERZANDE, Dirk, B-3740 Bilzen (BE); GELAN, Joanes, B-3600 Genk (BE); VAN BREEMEN, Albert, NL-5628 XA Eindhoven (NL); VAN DER BORGHT, Michael, B-2300 Turnhout (BE); LUTSEN, Laurence, F-59210 Coudekerque-Branche (FR); KIEBOOMS, Raf, B-3600 Genk (BE); KREUDER, Willi, D-55126 Mainz (DE)
(74) Vertreter: Luderschmidt, Schüler & Partner GbR
(86) Internationale Anmeldenummer: EP9906324
(87) Internationale Veröffentlichungsnummer: WO00014059

(56) Entgegenhaltungen:
- EP-A- 0 167 139
- EP-A- 0 170 073
- EP-A- 0 644 217
- EP-A- 0 705 857
- A. ISSARIS, ET AL.: "Polymerization mechanism of 1-[(butylsulphi(o)nyl)- methyl]-4-(halomethyl)benzene: The effect of polariser and leaving group" MACROMOLECULES, Bd. 31, Nr. 14, 14. Juli 1998 (1998-07-14), Seiten 4426-4431, XP000768801 American Chemical Society, Washington, DC, US ISSN: 0024-9297
- BURN P L ET AL: "Chemical tuning of the electronic properties of poly(p-phenylenevinylene)-based copolymers" JOURNAL OF THE AMERICAN CHEMICAL SOCIETY, Bd. 115, Nr. 22, 3. November 1993 (1993-11-03), Seiten 10117-10124, XP002088191 American Chemical Society, Washington, DC, US ISSN: 0002-7863 in der Anmeldung erwähnt
- P.M. LAHTI, ET AL.: "Polymerisation of alpha,alpha'-bis(dialkylsulphonio)- p-xylene dihalides via p-xylylene intermediates: evidence for a nonradical mechanism" JOURNAL OF THE AMERICAN CHEMICAL SOCIETY, Bd. 110, Nr. 21, 12. Oktober 1988 (1988-10-12), Seiten 7258-7259, XP002124196 American Chemical Society, Washington, DC, US ISSN: 0002-7863
- PATENT ABSTRACTS OF JAPAN vol. 014, no. 571 (C-0790), 19. Dezember 1990 (1990-12-19) & JP 02 250836 A (SANSHIN CHEMICAL INDUSTRY), 8. Oktober 1990 (1990-10-08)
- A.J.J.M. VAN BREEMEN, ET AL.: "Highly selective route for producing unsymmetrically substituted monomers toward synthesis of conjugated polymers derived from poly(p-phenylene vinylene)" JOURNAL OF ORGANIC CHEMISTRY, Bd. 64, Nr. 9, 30. April 1999 (1999-04-30), Seiten 3106-3112, XP002124197 American Chemical Society, Washington, DC, US ISSN: 0022-3263

## Beschreibung

Die vorliegende Erfindung betrifft ein Verfahren zur Herstellung von Derivaten des 4-Alkylsulfinylmethylarylenmethanols. Derivate des 4-Alkylsulfinylmethylarylenmethanols sind wertvolle Monomere in der Herstellung von konjugierten Polymeren, die unter anderem als Elektrolumineszenzmaterialien geeignet sind.

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeige- und Beleuchtungselementen, wie Glühlampen, Gasentladungslampen und nicht selbstleuchtenden Flüssigkristallanzeigeelementen, sind bereits seit einiger Zeit Elektrolumineszenz (EL)-materialien und -vorrichtungen, wie lichtemittierende Dioden (LED), in Gebrauch.

Neben anorganischen Elektrolumineszenzmaterialien und -vorrichtungen sind seit etwa 30 Jahren auch niedermolekulare, organische Elektrolumineszenzmaterialien und -vorrichtungen bekannt (siehe z.B. US-A-3,172,862). Bis vor kurzem waren aber solche Vorrichtungen in ihrer praktischen Anwendbarkeit stark eingeschränkt.

In der EP-A-0,423,283 und der EP-A-0,443,861 sind Elektrolumineszenzvorrichtungen beschrieben, die einen Film aus einem konjugierten Polymer als lichtemittierende Schicht (Halbleiterschicht) enthalten. Solche Vorrichtungen bieten zahlreiche Vorteile, wie die Möglichkeit, großflächige, flexible Displays einfach und kostengünstig herzustellen. Im Gegensatz zu Flüssigkristalldisplays sind Elektrolumineszenzdisplays selbstleuchtend und benötigen daher keine zusätzliche rückwärtige Beleuchtungsquelle.

Eine typische Vorrichtung nach EP-A-0,423,283 besteht aus einer lichtemittierenden Schicht in Form eines dünnen, dichten Polymerfilms (Halbleiterschicht), der mindestens ein konjugiertes Polymer enthält. Eine erste Kontaktschicht steht in Kontakt mit einer ersten Oberfläche, eine zweite Kontaktschicht mit einer weiteren Oberfläche der Halbleiterschicht. Der Polymerfilm der Halbleiterschicht hat eine genügend geringe Konzentration von extrinsischen Ladungsträgern, so daß beim Anlegen eines elektrischen Feldes zwischen den beiden Kontaktschichten Ladungsträger in die Halbleiterschicht eingebracht werden, wobei die eine Kontaktschicht positiv gegenüber der anderen wird, und die Halbleiterschicht Strahlung aussendet. Die in solchen Vorrichtungen verwendeten Polymere sind konjugiert. Unter konjugiertem Polymer versteht man ein Polymer, das ein delokalisiertes Elektronensystem entlang der Hauptkette besitzt. Das delokalisierte Elektronensystem verleiht dem Polymer Halbleitereigenschaften und gibt ihm die Möglichkeit, positive und/oder negative Ladungsträger mit hoher Mobilität zu transportieren.

In EP-A-0,423,283 und der EP-A-0,443,861 ist als polymeres Material für die lichtemittierende Schicht Poly(p-phenylenvinylen) beschrieben, welches mit Alkyl-, Alkoxy-, Halogen- oder Nitrosubstituenten am aromatischen Kern modifiziert werden kann. Derartige Polymere sind seither in einer großen Anzahl von Studien untersucht worden und gerade dialkoxysubstituierte PPVs sind schon sehr weit in Richtung Anwendungsreife hin optimiert worden (vgl. z. B. J. Salbeck, Ber. Bunsenges. Phys. Chem. 1996, 100, 1667). Allerdings kann die Entwicklung derartiger Polymere keinesfalls als abgeschlossen betrachtet werden. So sind unter anderem immer noch Verbesserungen hinsichtlich der Lebensdauer, der Beständigkeit und auch der erzielbaren Farbe nötig. Die am weitesten entwickelte obengenannte Polymerklasse, Dialkoxy-PPV, ist nämlich nur zur Emission orangeroten Lichtes geeignet.

Die vorstehenden Polymeren setzt einerseits hochreine Monomere als auch sehr komplexe Monomere voraus. Diese Monomerbausteine sind teilweise nur aufwendig in der erforderlichen Güte zugänglich und müssen nach ihrer aufwendigen Herstellung noch chromatographisch gereinigt werden.

Aus Macromolecules 31 (14) Seite 4426-31 sowie aus EP-A-0 644 217 und EP-A-0 705 857 sind Verfahren zur Herstellung von Verbindung der Formel (I) bekannt. Diese führen jedoch zu einem hohen Anteil an unerwinschten Neberprodukten welche aufwendij abgetrennt werden müssen.

Es besteht daher ein großer Bedarf an Verfahren zur Herstellung entsprechender Monomer-Bausteine, wobei die Monomere in einer solchen Qualität erhalten werden, daß auf eine chromatographische Reinigung verzichtet werden kann.

Gegenstand der vorliegenden Erfindung ist ein Verfahren zur Herstellung von Verbindungen der Formel (I) 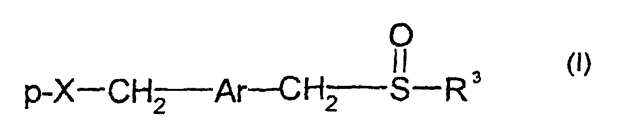 worin
- Ar: ein aromatisches Ringsystem mit 4 bis 20 Kohlenstoffatomen, der gegebenenfalls ein oder mehrfach mit C₁-C₂₀-Alkyl, C₃-C₂₀-Alkoxy, C₃-C₂₀-verzweigtem Alkyl, Phenyl oder Benzyl-Resten substituiert sein kann, und gegebenenfalls bis zu 4 Heteroatome aus der Gruppe Sauerstoff, Schwefel und Stickstoff im aromatischen Ringsystem enthalten kann,
- X: eine Abgangsgruppe, und
- R³: ein unverzweigtes Alkyl mit 1 bis 20 Kohlenstoffatomen, ein verzweigtes Alkyl mit 3 bis 20 Kohlenstoffatomen, ein cyclisches Alkyl, wie Cyclohexyl, oder ein C₁-C₄-alkylsubstituiertes cyclisches Alkyl, wie Cyclohexylmethyl, Phenyl oder Benzyl, die gegebenenfalls substituiert sein können, und/oder Heteroatome, wie O, N, Si enthalten können, bedeutet,
umfassend die Maßnahmen
a) Umsetzung einer Verbindung der Formel (II)

   p-X-CH₂-Ar-CH₂-X (II)

   worin Ar und X die in Formel (I) aufgezeigte Bedeutung haben, mit einem organischen Sulfid der Formel (III)

   R⁴-S-R⁵ (III)

   worin
   R⁴, R⁵ gleich oder verschieden ein unverzweigtes Alkyl mit 1 bis 20 Kohlenstoffatomen, ein verzweigtes Alkyl mit 3 bis 20 Kohlenstoffatomen, ein cyclisches Alkyl mit 3 bis 10 Kohlenstoffatomen wie Cyclobutyl, Cyclopentyl, Cyclohexyl, ein C₁-C₄-alkylsubstituiertes cyclisches Alkyl, wie Cyclohexylmethyl ist, oder R⁴ und R⁵ gemeinsam einen Ring bilden, der auch ein oder mehrere Heteroatome ausgewählt aus der Gruppe Sauerstoff, Schwefel und Stickstoff enthalten kann, bedeuten,
   zu einer Verbindung der Formel (IV) 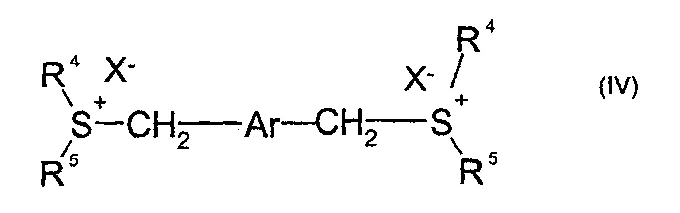 worin R⁴, R⁵, X und Ar die vorstehende Bedeutung haben,
b) Umsetzung der Verbindung der Formel (IV) mit 0,85 bis 1,1 Äquivalenten eines Mercaptans der Formel (V)

   R³-SH (V)

   worin
   - R3: ein unverzweigtes Alkyl mit 1 bis 20 Kohlenstoffatomen, ein verzweigtes Alkyl mit 3 bis 20 Kohlenstoffatomen, ein cyclisches Alkyl, wie Cyclohexyl, oder ein C₁-C₄-alkylsubstituiertes cyclisches Alkyl, wie Cyclohexylmethyl, Phenyl oder Benzyl, die gegebenenfalls substituiert sein können, und/oder Heteroatome, wie O, N, Si enthalten können, bedeutet,
   zu einer Verbindung der Formel (VI) 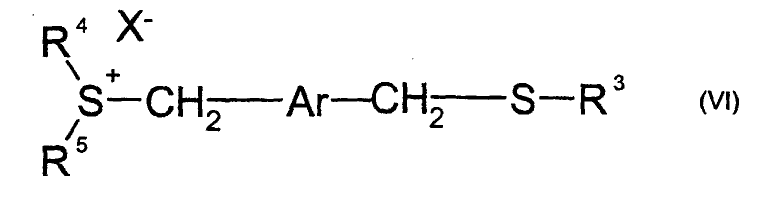
c) Erwärmen der Verbindung der Formel (VI) in einer Flüssigkeit und Bildung der Verbindung der Formel (VII) 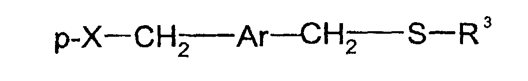 durch Abspaltung von einem organischen Sulfid der Formel (III), wobei die Verbindung der Formel (VII) in der vorstehenden Flüssigkeit gelöst vorliegt,
d) Oxidation der Verbindung der Formel (VII) mit einem Oxidationsmittel zur Verbindung der Formel (I).

Bevorzugt steht der Rest X für eine Abgangsgruppe wie Halogen, -O-Tosylat, -O-Mesylat oder -O-trifluoracetat.

In der Formel (I) steht p für para in Bezug auf die beiden Methylenreste -CH₂beiderseits des Restes Ar.

Bevorzugt werden mit dem erfindungsgemäßen Verfahren Verbindungen der Formel (I) in denen Ar für die Struktureinheit 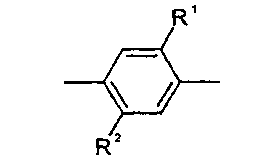 worin
- R¹, R²: gleich oder verschieden sind, und Wasserstoff, ein unverzweigter Alkyl- oder Alkoxy-Rest mit 1 bis 20 Kohlenstoffatomen, ein verzweigter Alkyl- oder Alkoxy-Rest mit 3 bis 20 Kohlenstoffatomen, Phenyl oder Benzyl, wobei die vorstehend genannten Reste gegebenenfalls substituiert sein können, Halogen, insbesondere Chlor, Brom oder Fluor, Cyano, Nitro, oder einen Ester mit 1 bis 20 Kohlenstoffatomen steht.

Insbesondere bevorzugt sind Verbindungen der Formel (I) in denen Ar die vorstehende Bedeutung hat und R¹ und R² unabhängig voneinander für die Reste unverzweigter Alkoxy-Rest mit 1 bis 20 Kohlenstoffatomen, ein verzweigter Alkoxy-Rest mit 3 bis 20 Kohlenstoffatomen, Phenyl oder Benzyl, wobei die vorstehend genannten Reste gegebenenfalls substituiert sein können, Halogen, insbesondere Chlor, Brom oder Fluor, Cyano, Nitro, oder einen Ester mit 1 bis 20 Kohlenstoffatomen steht.
Weiterhin bevorzugt sind Verbindungen der Formel (I) in denen R¹ und R² unabhängig voneinander für die Reste unverzweigter Alkoxy-Rest mit 1 bis 10 Kohlenstoffatomen, ein verzweigter Alkoxy-Rest mit 3 bis 20 Kohlenstoffatomen, Phenyl, der durch ein oder mehrere, verzweigte oder unverzweigte Alkyl- oder Alkoxygruppen mit bis zu 20 C-Atomen substituiert sein kann, steht.
Bevorzugt steht R³ für n-, i-,s-, t-Butyl, i-Pentyl, Octyl, 3,6,9,-trioxadecyl, 2-Hydroxyethyl, 2-Chlorethyl, besonders bevorzugt n-Butyl und n-Octyl.

Gleichermaßen bevorzugt werden mit dem erfindungsgemäßen Verfahren Verbindungen der Formel (I) in denen Ar für die Struktureinheit 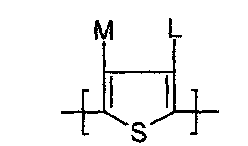 worin
- M, L: gleich oder verschieden sind, und Wasserstoff, ein unverzweigter Alkyl- oder Alkoxy-Rest mit 1 bis 16 Kohlenstoffatomen, ein verzweigter Alkyl- oder Alkoxy-Rest mit 3 bis 16 Kohlenstoffatomen, Phenyl oder Benzyl, wobei die vorstehend genannten Reste gegebenenfalls substituiert sein können, Halogen, insbesondere Chlor, Brom oder Fluor, Cyano, Nitro, oder einen Ester mit 1 bis 16 Kohlenstoffatomen steht, oder M und L gemeinsam eine Brücke mit mindestens 4 Brückengliedem, die auch ein oder mehrere Heteroatome, insbesondere Sauerstoff und/oder Schwefel, enthalten können.

Die Umsetzung der Verbindung der Formel (II) mit einem Sulfid der Formel (III) unter Ausbildung des Bissulfoniumsalzes der Formel (IV) ist beispielsweise in P.L.Burn, D.D.Bradley, R.H. Friend, D.A.Halliday, A.B.Holmes, J.Chem.Soc.Perkin Trans. 1,23,1992, 3225; P.L. Bum et al., J.Amer.Chem.Soc., 115, 22, 1993, 10117; Lee et al., Mol.Cryst.Liq.Cryst.Sci.Technol.Sect.A, 247, 1994, 121; D. Hwang, J.Chem.Soc.Chem.Commun. 21,1994, 2461; J. Jin et al., J.Chem.Soc.Chem.Commun. 17,1989, 1205; J.D. Stenger-Smith et al., J.Org.Chem. 59, 20,1994, 6107; Hwang, C. Yoon, K. Moon, H. Shim, Mol.Cryst.Liq.Cryst.Sci.Technol.Sect.A, 280, 1996, 39 und 175 und 181; A. Luettringhaus, H. Machatzke, Arzneimittel Forsch. 13, 1963, 366; Bardsley, Ashford, Biochem. J. 128, 1972, 253-6; Luettringhaus, H. Machatzke, Justus Liebigs Ann.Chem. 671, 1964, 165-196; G.M. Brooke, S.D. Mawson, J.Fluorine Chem. 50, 1, 1990, 101; S. Antoun, Collect.Czech.Chem.Commun. 52, 1, 1987, 162 oder Y. Sonoda, K. Kaeriyama, Bull.Chem.Soc.Jpn. 65, 3, 1992, 853 beschrieben.

Bei dem organischen Sulfid der Formel (III) sind Dimethylsulfid , Diethylsulfid, 2-Ethylthioethanol, Thiobisethanol, oder ein cyclisches Sulfid, wie Tetrahydrothiopyran (=Pentamethylensulfid) oder Tetrahydrothiophen (THT) bevorzugt. Insbesondere bevorzugt ist Tetrahydrothiophen (THT).

Üblicherweise erfolgt die Umsetzung in Lösungsmitteln wie Methanol, Wasser, Ethanol, Aceton, Dioxan, Tetrahydropyran, Tetrahydrofuran, Acetonitril, wobei häufig auch Gemische vorteilhaft sind, bei Temperaturen zwischen Raumtemperatur (20°C) und Siedetemperatur des Gemisches (ca. 100°C), bevorzugt zwischen 20° und 60°C.

Pro Mol Ausgangssubstanz der Formel (II) werden 2 bis 8 Äquivalente Sulfid der Formel (III) eingesetzt. Bevorzugt werden ca. 5 Äquivalente eingesetzt.
Im nachfolgenden Reaktionsschritt (b) wird das Bissulfoniumsalz der Formel (IV) mit Mercaptan der Formel (V) umgesetzt.

Die Umsetzung erfolgt in Gegenwart einer Base, wobei pro Mol Bissulfoniumsalz 0,85 bis 1,1 Äquivalente Base, insbesondere 0,9 bis 1,05 Äquivalente, zugesetzt werden. Geeignete Basen sind anorganische oder organische Basen. Geeignete anorganische Basen sind NaOH, KOH und LiOH. Geeignete organische Basen sind insbesondere sterisch gehinderte, wie Lithiumdiisopropylamid (LDA), Natriumtrimethylsilanoat, Bis(trimethylsilyl)kaliumamid, insbesondere jedoch Alkali-tert. Butanolate, wie KOtBu und NaOtBu.

Pro Mol Bissulfoniumsalz der Formel (IV) werden 0,85 bis 1,1 Äquivalente Mercaptan der Formel (V), insbesondere 0,95 bis 1,05 Äquivalente, eingesetzt. Die Umsetzung erfolgt vorteilhafterweise bei Temperaturen zwischen 0° und 40°C, inbesondere bevorzugt unterhalb Raumtemperatur (ca. 10°C). Die Umsetzung erfolgt in Gegenwart eines polaren, protischen Lösungsmittels. Geeignete Lösungsmittel sind Ethanol, n- und i-Propanol, n-,i-, sec., tert.-Butanol und Gemische, insbesondere Methanol.

Anschließend wird das Reaktionsgemisch neutralisiert, aufkonzentriert, ggfs. von Feststoff befreit und mit einem unpolarem, aprotischen organischen Lösungsmittel verdünnt, dessen Siedepunkt oberhalb des vorstehend genannten polaren, protischen Lösungsmittel liegt. Die leichtersiedende Fraktion aus polarem, protischen Lösungsmittel und Sulfid wird anschließend, beispielsweise durch Verdampfen bei reduziertem Druck, oder andere geeignete Maßnahmen entfernt. Durch Wiederholen dieses Vorgangs wird das Sulfid der Formel (III) aus dem Gleichgewicht herausgeschleppt und durch die Abgangsgruppe X ersetzt.

Die verbleibende, konzentrierte Lösung enthält neben der Verbindung der Formel (VI) auch noch die beiden möglichen Nebenprodukte der Formel (A)

R³-S-CH₂-Ar-CH₂-S-R³ (A)

und der Formel (B)

X-CH₂-Ar-CH₂-X (B)

Überraschender Weise beträgt der Anteil an Nebenprodukten der Formel (A) und (B) - nicht wie erwartet - jeweils 25% der Theorie, sondern deutlich weniger. Die nachgewiesenen Anteile von Verbindungen der Formel (A) und (B) betragen überraschenderweise weniger als 15% und oft nur 5%.

Die so erhaltene Verbindung der Formel (VII) wird anschließend mittels literaturbekannter Methoden zum Sulfoxyd oxidiert (J. March, Adv. Org. Chem. 3^{rd} Ed., J. Wiley, New York, p.1089-90).

Bevorzugt wird eine katalytische Oxidation mit Peroxiden, insbesondere Wasserstoffperoxid. Geeignete Katalysatoren sind beispielsweise SeO₂ und TeO₂, Luft oder Sauerstoff läßt sich mit katalytischen Mengen an NO₂ auch vorteilhaft einsetzen.

Die Isolierung der Zielverbindung der Formel (I) erfolgt vorteilhafter Weise durch Umkristallisation aus niedrig siedenden Lösungsmitteln wie Alkanen, Alkoholen, Ketonen, Ethern oder Estern. Eine aufwendige chromatographische Reinigung ist aufgrund der hohen Selektivität deserfindungsgemäßen Verfahrens nicht erforderlich.

Weiterer Gegenstand der vorliegenden Erfindung sind die Verbindungen der Formel (VI) 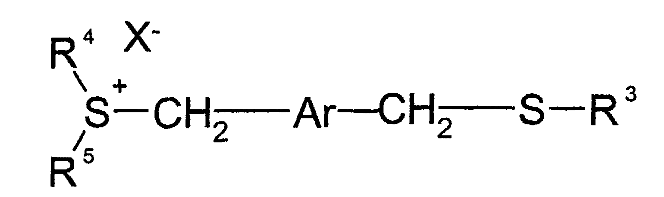 worin Ar, R³, R⁴, R⁵ und X die vorstehend genannte Bedeutung haben.

Die Verbindungen der Formel (VI) sind wertvolle Zwischenprodukte in der Synthese von Verbindungen der Formel (I) und können gegebenenfalls isoliert werden.

### Beispiele

### Beispiel 1: p-Xylylenbis(tetrahydrothiopheniumchlorid)

Eine Lösung von 52,5 g (0,3 mol) p-Xylylendichlorid in 105 ml (1,19 mol) Tetrahydrothiophen und 105 ml Methanol wurde 60 h bei Raumtemperatur gerührt.

Das Reaktionsgemisch wurde bei -10°C in 420 ml Aceton eingerührt. Der hygroskopische Niederschlag wurde mit 600 ml kaltem Aceton nachgewaschen und getrocknet 95,9 g , 91 %.

### Beispiel 2: p-(Octylthiomethyl)benzylchlorid

1,83 g (19 mmol) Natrium-t-butanolat und 2,78 g (19 mmol) Octanthiol wurden bei Raumtemperatur in 40 g Methanol gerührt. Nach 30 min wurde die klare Lösung in einer Portion zu 6,68 g (19 mmol) des p-Xylylenbis(tetrahydrothiophenium-chlorid)s, erhalten gemäß Beispiel 1, in 100 g Methanol gegeben. Nach einer Stunde wurde mit 1N Salzsäure neutralisiert und alles Flüchtige im Vakuum entfernt. Das Rohprodukt wurde in 200 ml Chloroform aufgenommen, Ungelöstes abfiltriert und das Filtrat im Vakuum eingeengt. Das Öl wurde mit dem doppelten Volumen Octan vermischt und zusammen mit Tetrahydrothiophen im Vakuum abdestilliert. Diese Sequenz wurde noch dreimal mit Octan wiederholt und schließlich 5,31 g erhalten, bestehend aus 90 % Produkt, 5 % Edukt und 5 % p-Bis(octylthiomethyl)benzol. Diese Reaktion wurde bei verschiedenen Temperaturen durchgeführt und die Produktverteilung ¹H-NMR-spektroskopisch analysiert (alle Angaben in mol-%).

| Temp./°C | Edukt | Produkt | p-Bis(octylthiomethyl)benzol |
|---|---|---|---|
| -20 | 8 | 84 | 8 |
| 0 | 6 | 87 | 7 |
| 20 | 5 | 90 | 5 |
| 40 | 6 | 87 | 7 |

Wiederholung der Versuche bei 20°C, jedoch mit geringem Unterschuß des Mercaptans ergab folgende Produktverteilung:

| RSH-Äquivalente | Edukt | Produkt | p-Bis(octylthiomethyl)benzol |
|---|---|---|---|
| 0,95 | 14 | 78 | 8 |
| 0,98 | 8 | 84 | 8 |
| 1,00 | 5 | 90 | 5 |

### Vergleichsbeispiel A: p-(Octylthiomethyl)benzylchlorid

Zu einer gerührten Mischung von 39,4 g (0,225 mol) p-Xylylendichlorid in 400 ml Toluol, 23,7 g (0,59 mol) NaOH in 400 ml Wasser und 1 g Aliquat 336 als Phasentransferkatalysator wurde über 24 h eine Lösung von 14,3 g (0,10 mol) Octanthiol in 100 ml Toluol bei Raumtemperatur zugetropft. Die abgetrennte organische Schicht wurde dreimal mit je 200 ml Wasser gewaschen, getrocknet und im Vakuum eingeengt. 32,0 g, das ¹H-NMR-spektroskopisch bestand aus 39 % Produkt, 59 % Edukt und 2 % p-Bis(octylthiomethyl)benzol)
Wiederholung des Versuchs, jedoch ohne Überschuß des p-Xylylendichlorids ergab folgende Produktverteilung:

| RHS-Äquivalente | Edukt | Produkt | p-Bis(octylthiomethyl)benzol) |
|---|---|---|---|
| 1,00 | 19-25 | 50-63 | 18-25 |
| 0,45 | 70 | 28 | 2 |

### Vergleichsbeispiel B: p-(Octylthiomethyl)benzylchlorid

1,83 g (19 mmol) Natrium-t-butanolat und 2,78 g (19 mmol) Octanthiol wurden bei Raumtemperatur in 40 g Methanol gerührt. Nach 30 min wurde die klare Lösung in einer Portion zu 3,33 g (19 mmol) p-Xylylendichlorid in 100 g Methanol gegeben. Nach einer Stunde wurde mit 1n Salzsäure neutralisiert uns alles Flüchtige im Vakuum entfernt. Das Rohprodukt wurde in 250 ml Chloroform aufgenommen und zweimal mit je 100 ml Wasser gewaschen, getrocknet und im Vakuum eingeengt. 5,29 g, das 1H-NMR-spektroskopisch bestand aus 50 % Produkt, 25 % Edukt und 25 % p-Bis(octylthiomethyl)benzol.

### Beispiel 3: p-(Butylsulfinylmethyl)benzylchlorid

Eine Lösung von 17,1 g (178 mmol) Natrium-t-butanolat und 19,4 ml (16,4 g, 181 mmol) Butanthiol in Methanol wurde zu einer klaren Lösung von 67,1 g des Bissulfoniumsalzes in 300 ml Methanol, hergestellt gemäß Beispiel 1, zugegeben. Nach 30 min wurde das Lösungsmittel entfernt, Octan zugegeben und abdestilliert. Diese letzte Sequenz wurde dreimal wiederholt. Das zurückbleibende Öl wurde in Chloroform aufgenommen, mit Wasser und Natriumhydrogencarbonat-Lösung gewaschen, über Magnesiumsulfat getrocknet und filtriert, wodurch auch ungelöste organische Reste entfernt wurden. Zu einer Lösung dieses Zwischenprodukts in 50 ml 1,4-Dioxan, 250 ml Methanol und Tellurdioxid wurde Wasserstoffperoxid zugesetzt. Nach fünf Stunden wurden 100 ml gesättigte Kochsalzlösung hinzugefügt, und die Mischung mit Chloroform extrahiert, getrocknet, und 43,4 g p-(Butylsulfinylmethyl)benzylchlorid, erhalten.
Durch Umkristallisation aus 800 ml Aceton, gefolgt von einer Kristallisation aus Chloroform / Hexan wurden 40 g des Produkts in höchster Reinheit erhalten. ¹H-NMR-spektroskopisch sind Verunreinigungen nur noch auf dem Niveau von ¹³C-Satelitten erkennbar.

### Beispiel 4:

2-(3,7-Dimethyloctyloxy)-5-methoxy-xylylen-1,4-bis(tetrahydrothiopheniumchlorid) 10 g (27,7 mmol) 1,4-Bischlormethyl-2-(3,7-dimethyloctyloxy)-5-methoxy-benzol (BCDM) und 12,4 g (141 mmol) Tetrahydrothiophen (THT) wurden gelöst in 20 ml Methanol bei 30°C gerührt. Nach 70 h wurde überschüssiges THT abdestilliert und der Rückstand in einer gerade ausreichenden Menge Methanol gelöst, filtriert, und in 100 ml eiskaltes Aceton eingerührt. Der Feststoff wurde mit 40 ml Hexan digeriert. Nach 4 h Trocknen im Vakuum wurden 11 g (72 % d. Th.) erhalten.

### Beispiel 5:

2-(3,7-Dimethyloctyloxy)-5-methoxy-4-butylthiomethyl-benzylchlorid über 2-(3,7-Dimethyloctyloxy)-5-methoxy-4-butylthlomethyl-benzyl tetrahydrothiopheniumchlorid, jeweils als Gemische der Regioisomere
6,5 g (68 mmol) Natrium-t-butanolat (1 Äq.) und 6,0 g (66,5 mmol, 0,98 Äq,) Butanthiol (BuSH) wurden in 150 ml Methanol bei 20°C gerührt. Nach 30 min wurde die klare Lösung tropfenweise zu einer Lösung von 37,6 g (68 mmol, 1 Äq.) des Salzes, hergestellt gemäß Beispiel 4, in 400 ml Methanol zugegeben. Die Mischung wurde nach einer Reaktionszeit von 60 min bei 20°C mit 1n Salzsäure neutralisiert, woraufhin alles Flüchtige im Vakuum abdestilliert wurde. Der Rückstand wurde in 200 ml Chloroform aufgenommen und filtriert. Das Filtrat wurde im Vakuum aufkonzentriert und das erhaltene Öl in 80 ml aufgenommen. Nach Verdampfen des Gemischs aus Octan und THT wird dieser Vorgang noch dreimal wiederholt. Ausbeute 39,5 g, bestehend aus 18 % BCDM, 7 % Bisthioether und 74 % des gewünschten Produkts, als 50:50 Gemisch der beiden Regioisomere 2-(3,7-Dimethyloctyloxy)-5-methoxy-4-butylthiomethyl-benzylchlorid und 5-(3,7-Dimethyloctyloxy)-2-methoxy-4-butylthiomethyl-benzylchlorid. Beide Regioisomere wurden auch durch präparative Säulenchromatographie isoliert.

### Beispiel 6:

Beispiel 5 wurde wiederholt mit folgenden Änderungen:
a. Reaktionszeit 40 statt 60 min.
b. 1,1 Äquivalente NaOtBu statt 1 Äq.
c. 0,99 Äquivalente BuSH statt 0,98 Äq.
Erhalten wurden16 % BCDM, 7 % Bisthioether und 77 % des gewünschten Produkts als 49:51 Gemisch der beiden Regioisomere, und geringe Spuren des hier unerwünschten Polymeren.

### Beispiel 7:

39,5 g des Produkts gemäß Beispiel 5 wurden in 200 ml Dioxan gelöst und mit 1,82 g (11,4 mmol, 12 mol-%) Tellurdioxid versetzt. Unter heftigem Rühren wurden bei Raumtemperatur 18,5 g (190 mmol) einer 35 %-igen wäßrigen WasserstoffperoxidLösung zugetropft. Sobald dünnschicht-chromatographisch eine Spur des Sulfons als Zeichen der Überoxidation zu erkennen war (3,5 h), wurde die Reaktion durch Eingießen in Eiswasser abgebrochen. Die wäßrige Phase wurde mit drei Portionen Chloroform extrahiert (250; 100; 100 ml). Die vereinigten organischen Phasen ergaben nach Trocknung 30 g des Produktes: 76% der erwünschten Regioisomere 2-(3,7-Dimethyloctyloxy)-5-methoxy-4-butylsulfinylmethyl-benzylchlorid und 5-(3,7-Dimethyloctyloxy)-2-methoxy-4-butylsulfinylmethyl-benzylchlorid, 16 % BCDM und 7% des Bis(butylsulfinyl)-Derivates.
Dieses Gemisch läßt sich bereits ohne Reinigung erfolgreich zur Polymerisation einsetzen.

### Beispiel 8

Beispiel 5 wurde wiederholt, wobei die Reaktionstemperatur von 20° auf 10° bzw. 0°C gesenkt wurde. Die folgende Produktverteilung wurde gefunden;

| Temp./°C | BCDMBisbutylthio-Derivat | | Produkt (Regioisomere) | |
|---|---|---|---|---|
| 20 | 18 | 7 | 74 | (50:50) |
| 10 | 19 | 4 | 77 | (51:49) |
| 0 | 17 | 4 | 79 | (51:49) |

### Beispiel 9:

Analog zu Beispiel 5 wurden 2,5-Dimethyl-xylylendichlorid (9a), 2,5-Dimethoxy-xylylendichlorid (9b), und 2,5-Dichlor-xylylendichlorid (9c), wobei wegen der verbesserten Löslichkeit Methanol bzw. Mischungen mit Wasser als Lösungsmittel bei der Reaktion mit BuSH ausgewählt wurde umgesetzt;

| Bsp. | MeOH:H₂O | Edukt | Produkt | Bis(butylthlo)xylol-Derivat |
|---|---|---|---|---|
| 9a | 1:0 | 9 | 86 | 5 |
| 9b | 1:0 | 8 | 80 | 12 |
| 9c | 4:1 | 7 | 86 | 7 |

Auf analoge Weise läßt sich 4-Butylthiomethyl-2,5-dicyano-benzylchlorid in Acetonitril : Wasser (65:35) herstellen. In wasserfreiem Methanol wurde 2-Butylthiomethyl-6-chlormethyl-naphthalin erhalten.

### Vergleichsversuch: p-Phenylsulfinylmethylbenzylchlorid

3,8 ml (37,8 mmol) Thiophenol wurden in 100 ml trockenem Tetrahydrofuran (THF) gelöst, und langsam 0,91 g (37,8 mmol) NaH dazu gegeben. Die Suspension wurde in eine bei 25°C kräftig gerührte Lösung von 33,08 g (189 mmol, d.h. fünffachem Überschuß) p-Bischlormethylbenzol in 150 ml eingetropft. Nach 14 h wurden 100 ml Wasser zugegeben und viermal mit je 50 ml Chloroform extrahiert. Die vereinigten organischen Phasen wurden über Magnesiumsulfat getrocknet und das Lösungsmittel verdampft, zuletzt im Vakuum.
Das rohe Gemisch bestehend aus p-Bischlormethylbenzol, p-(Bisphenylthiomethyl)benzol und dem gewünschten p-Phenylthiomethylbenzylchlorid wurde ohne weitere Reinigung der Oxidation zum Sulfoxid unterworfen:
Das Gemisch wurde zusammen mit 0,6 g (0,037 mmol) TeO₂ in 450 ml Methanol gelöst. 8,6 ml 30-Gew.-%igem Wasserstoffperoxid (76 mmol) wurden unter gutem Rühren zugetropft. 4 Stunden später wurden 100 ml Wasser zugegeben, die wäßrige Phase mit Chloroform extrahiert, wie üblich mit Magnesiumsulfat getrocknet, und zur Trockne eingeengt. Das Rohprodukt (30 g) wurde über Silicagel (300 g) chromatographisch gereinigt. Nach Umkristallisation aus Hexan/Toluol wurden 5,5 g p-Phenylsulfinylmethylbenzylchlorid als farblose Kristalle erhalten. Die Ausbeute beträgt 55 % bezüglich Thiophenol, aber nur 11 % bezüglich der Bischlormethylverbindung (5-facher Überschuß).

## Patentansprüche

1. Verfahren zur Herstellung von Verbindungen der Formel (I) 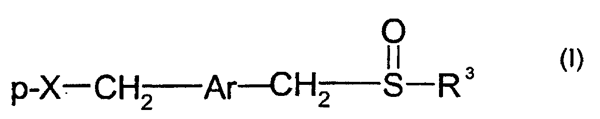 worin
Ar ein aromatisches Ringsystem mit 4 bis 20 Kohlenstoffatomen, der gegebenenfalls ein oder mehrfach mit C₁-C₂₀-Alkyl, C₃-C₂₀-Alkoxy, C₃-C₂₀-verzweigtem Alkyl, Phenyl oder Benzyl-Resten substituiert sein kann, und gegebenenfalls bis zu 4 Heteroatome aus der Gruppe Sauerstoff, Schwefel und Stickstoff im aromatischen Ringsystem enthalten kann,
X eine Abgangsgruppe, und
R³ ein unverzweigtes Alkyl mit 1 bis 20 Kohlenstoffatomen, ein verzweigtes Alkyl mit 3 bis 20 Kohlenstoffatomen, ein cyclisches Alkyl, oder ein C₁-C₄-alkylsubstituiertes cyclisches Alkyl, Phenyl oder Benzyl, die gegebenenfalls substituiert sein können, und/oder Heteroatome, enthalten können, bedeutet,
umfassend die Maßnahmen
a) Umsetzung einer Verbindung der Formel (II)
p-X-CH₂-Ar-CH₂-X (II)
worin Ar und X die in Formel (I) aufgezeigte Bedeutung haben, mit einem organischen Sulfid der Formel (III)
R⁴-S-R⁵ (III)
worin
R⁴, R⁵ gleich oder verschieden ein unverzweigtes Alkyl mit 1 bis 20 Kohlenstoffatomen, ein verzweigtes Alkyl mit 3 bis 20 Kohlenstoffatomen, ein cyclisches Alkyl mit 3 bis 10 Kohlenstoffatomen, ein C₁-C₄-alkylsubstituiertes cyclisches Alkyl, ist, oder R⁴ und R⁵ gemeinsam einen Ring bilden, der auch ein oder mehrere Heteroatome ausgewählt aus der Gruppe Sauerstoff, Schwefel und Stickstoff enthalten kann,
zu einer Verbindung der Formel (IV) 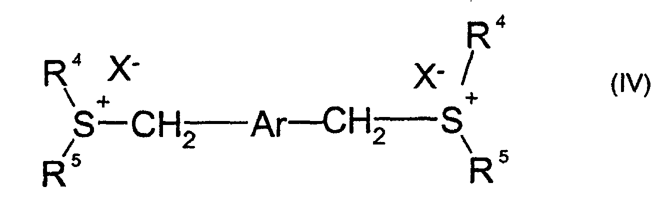 worin R⁴, R⁵, X und Ar die vorstehende Bedeutung haben,
b) Umsetzung der Verbindung der Formel (IV) mit 0,85 bis 1,1 Äquivalenten eines Mercaptans der Formel (V)
R³-SH (V)
worin
R³ ein unverzweigtes Alkyl mit 1 bis 20 Kohlenstoffatomen, ein verzweigtes Alkyl mit 3 bis 20 Kohlenstoffatomen, ein cyclisches Alkyl, oder ein C₁-C₄-alkylsubstituiertes cyclisches Alkyl, Phenyl oder Benzyl, die gegebenenfalls substituiert sein können, und/oder Heteroatome, enthalten können, bedeutet,
zu einer Verbindung der Formel (VI) 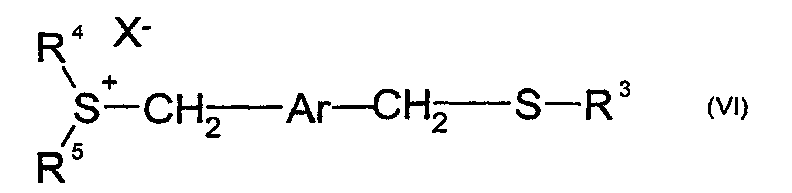
c) Erwärmen der Verbindung der Formel (VI) in einer Flussigkeit und Bildung der Verbindung der Formel (VII) 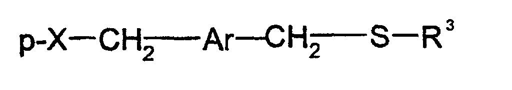 durch Abspaltung von einem organischen Sulfid der Formel (III), wobei die Verbindung der Formel (VII) in der vorstehenden Flüssigkeit gelöst vorliegt,
d) Oxidation der Verbindung der Formel (VII) mit einem Oxidationsmittel zur Verbindung der Formel (I).

2. Verfahren gemäß Anspruch 1, **dadurch gekennzeichnet, daß** der Rest X für eine Abgangsgruppe Halogen, -O-Tosylat, -O-Mesylat oder -O-trifluoracetat steht.

3. Verfahren gemäß Anspruch 1 oder 2, **dadurch gekennzeichnet, daß** Ar für die Struktureinheit 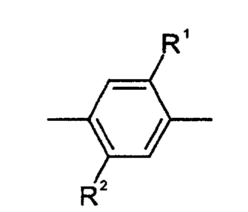 worin
R¹, R² gleich oder verschieden sind, und Wasserstoff, ein unverzweigter Alkyl- oder Alkoxy-Rest mit 1 bis 20 Kohlenstoffatomen, ein verzweigter Alkyl- oder Alkoxy-Rest mit 3 bis 20 Kohlenstoffatomen, Phenyl oder Benzyl, wobei die vorstehend genannten Reste gegebenenfalls substituiert sein können, Halogen, Cyano, Nitro, oder einen Ester mit 1 bis 20 Kohlenstoffatomen steht.

4. Verfahren gemäß einem der Ansprüche 1 bis 3, **dadurch gekennzeichnet, daß** Ar für die Struktureinheit 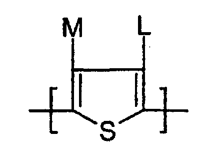 worin
M, L gleich oder verschieden sind, und Wasserstoff, ein unverzweigter Alkyl- oder Alkoxy-Rest mit 1 bis 16 Kohlenstoffatomen, ein verzweigter Alkyl- oder Alkoxy-Rest mit 3 bis 16 Kohlenstoffatomen, Phenyl oder Benzyl, wobei die vorstehend genannten Reste gegebenenfalls substituiert sein können, Halogen, Cyano, Nitro, oder einen Ester mit 1 bis 16 Kohlenstoffatomen steht, oder M und L gemeinsam eine Brücke mit mindestens 4 Brückengliedem, die auch ein oder mehrere Heteroatome, enthalten können, steht.

5. Verfahren gemäß Anspruch 3, **dadurch gekennzeichnet, daß** R¹ und R² unabhängig voneinander für die Reste unverzweigter Alkoxy-Rest mit 1 bis 20 Kohlenstoffatomen, ein verzweigter Alkoxy-Rest mit 3 bis 20 Kohlenstoffatomen, Phenyl oder Benzyl, wobei die vorstehend genannten Reste gegebenenfalls substituiert sein können, Halogen, Cyano, Nitro, oder einen Ester mit 1 bis 20 Kohlenstoffatomen steht.

6. Verfahren gemäß Anspruch 5, **dadurch gekennzeichnet, daß** R¹ und R² unabhängig voneinander für die Reste unverzweigter Alkoxy-Rest mit 1 bis 10 Kohlenstoffatomen, ein verzweigter Alkoxy-Rest mit 3 bis 20 Kohlenstoffatomen, Phenyl, der durch ein oder mehrere, verzweigte oder unverzweigte Alkyl- oder Alkoxygruppen mit bis zu 20 C-Atomen substituiert sein kann, steht.

7. Verfahren gemäß einem der Ansprüche 1 bis 6, **dadurch gekennzeichnet, daß** R³ für n-, i-,s-, t-Butyl, i-Pentyl, Octyl, 3,6,9,-trioxadecyl, 2-Hydroxyethyl oder 2-Chlorethyl steht.

8. Verfahren gemäß einem der Ansprüche 1 bis 7, **dadurch gekennzeichnet, daß** als organisches Sulfid der Formel (III) Dimethylsulfid , Diethylsulfid, 2-Ethylthioethanol, Thiobisethanol, oder ein cyclisches Sulfid, eingesetzt wird.

9. Verfahren gemäß einem der Ansprüche 1 bis 8, **dadurch gekennzeichnet, daß** die Umsetzung gemäß Maßnahme (a) in Methanol, Wasser, Ethanol, Aceton, Dioxan, Tetrahydropyran, Tetrahydrofuran, Acetonitril, oder Gemische derselben, durchgeführt wird.

10. Verfahren gemäß einem der Ansprüche 1 bis 9, **dadurch gekennzeichnet, daß** die Umsetzung gemäß Maßnahme (a) bei Temperaturen zwischen Raumtemperatur (20°C) und Siedetemperatur des Gemisches (100°C) durchgeführt wird.

11. Verfahren gemäß einem der Ansprüche 1 bis 10, **dadurch gekennzeichnet, daß** die Umsetzung gemäß Maßnahme (b) in Gegenwart einer Base erfolgt.

12. Verfahren gemäß einem der Ansprüche 1 bis 11, **dadurch gekennzeichnet, daß** pro Mol der Verbindung der Formel (IV) 0,95 bis 1,05 Äquivalente Mercaptan der Formel (V) eingesetzt werden.

13. Verfahren gemäß einem der Ansprüche 1 bis 12, **dadurch gekennzeichnet, daß** die Umsetzung gemäß Maßnahme (b) zwischen 0°C und 40°C erfolgt.

14. Verfahren gemäß einem der Ansprüche 1 bis 13, **dadurch gekennzeichnet, daß** die Umsetzung gemäß Maßnahme (b) in einem polaren, protischen Lösemittel erfolgt.

15. Verbindungen der Formel (VI) 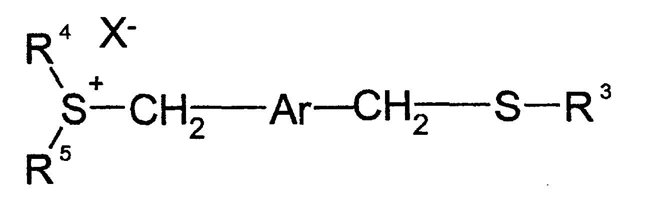 worin
Ar ein aromatisches Ringsystem mit 4 bis 20 Kohienstoffatomen, der gegebenenfalls ein oder mehrfach mit C₁-C₂₀-Alkyl, C₃-C₂₀-Alkoxy, C₃-C₂₀-verzweigtem Alkyl, Phenyl oder Benzyl-Resten substituiert sein kann, und gegebenenfalls bis zu 4 Heteroatome aus der Gruppe Sauerstoff, Schwefel und Stickstoff im aromatischen Ringsystem enthalten kann,
X eine Abgangsgruppe, und
R³ ein unverzweigtes Alkyl mit 1 bis 20 Kohlenstoffatomen, ein verzweigtes Alkyl mit 3 bis 20 Kohlenstoffatomen, ein cyclisches Alkyl, oder ein C₁-C₄-alkylsubstituiertes cyclisches Alkyl, Phenyl oder Benzyl, die gegebenenfalls substituiert sein können, und/oder Heteroatome, enthalten können, bedeutet,
R⁴, R⁵ gleich oder verschieden ein unverzweigtes Alkyl mit 1 bis 20 Kohlenstoffatomen, ein verzweigtes Alkyl mit 3 bis 20 Kohlenstoffatomen, ein cyclisches Alkyl mit 3 bis 10 Kohlenstoffatomen ein C₁-C₄-alkylsubstituiertes cyclisches Alkyl, ist, oder R⁴ und R⁵ gemeinsam einen Ring bilden, der auch ein oder mehrere Heteroatome ausgewählt aus der Gruppe Sauerstoff, Schwefel und Stickstoff enthalten kann, bedeuten,

16. Verbindungen gemäß Anspruch 15, **dadurch gekennzeichnet, daß** Ar für die Struktureinheit 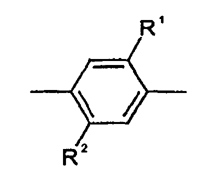 worin
R¹, R² gleich oder verschieden sind, und Wasserstoff, ein unverzweigter Alkyl- oder Alkoxy-Rest mit 1 bis 20 Kohlenstoffatomen, ein verzweigter Alkyl- oder Alkoxy-Rest mit 3 bis 20 Kohlenstoffatomen, Phenyl oder Benzyl, wobei die vorstehend genannten Reste gegebenenfalls substituiert sein können, Halogen, Cyano, Nitro, oder einen Ester mit 1 bis 20 Kohlenstoffatomen, steht.

17. Verbindungen gemäß Anspruch 15, **dadurch gekennzeichnet, daß** Ar für die Struktureinheit 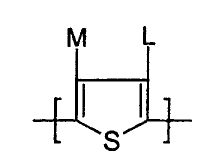 worin
M, L gleich oder verschieden sind, und Wasserstoff, ein unverzweigter Alkyl- oder Alkoxy-Rest mit 1 bis 16 Kohlenstoffatomen, ein verzweigter Alkyl- oder Alkoxy-Rest mit 3 bis 16 Kohlenstoffatomen, Phenyl oder Benzyl, wobei die vorstehend genannten Reste gegebenenfalls substituiert sein können, Halogen, Cyano, Nitro, oder einen Ester mit 1 bis 16 Kohlenstoffatomen steht, oder M und L gemeinsam eine Brücke mit mindestens 4 Brückengliedern, die auch ein oder mehrere Heteroatome, enthalten können, steht.

## Claims

1. A process for the preparation of compounds of the formula (I) 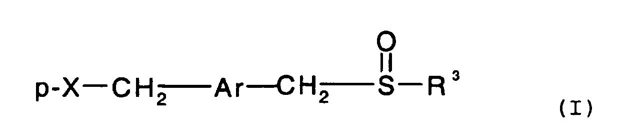 in which
Ar is an aromatic ring system having 4 to 20 carbon atoms, which may, if desired, be monosubstituted or polysubstituted by C₁-C₂₀-alkyl, C₃-C₂₀-alkoxy, C₃-C₂₀-branched alkyl, phenyl or benzyl radicals and which may contain up to 4 heteroatoms from the group consisting of oxygen, sulfur and nitrogen in the aromatic ring system,
X is a leaving group, and
R³ is unbranched alkyl having 1 to 20 carbon atoms, branched alkyl having 3 to 20 carbon atoms, cyclic alkyl or C₁-C₄-alkyl-substituted cyclic alkyl phenyl or benzyl, which may be substituted or unsubstituted and/or contain heteroatoms,
comprising the following measures:
a) reaction of a compound of the formula (II)
p-X-CH₂-Ar-CH₂-X (II)
in which Ar and X are as defined under the formula (I), with an organic sulfide of the formula (III)
R⁴-S-R⁵ (III)
in which
R⁴ and R⁵ are identical or different and are unbranched alkyl having 1 to 20 carbon atoms, branched alkyl having 3 to 20 carbon atoms, cyclic alkyl having 3 to 10 carbon atoms or C₁-C₄-alkyl-substituted cyclic alkyl or R⁴ and
R⁵ together form a ring, which may also contain one or more heteroatoms selected from the group consisting of oxygen, sulfur and nitrogen,
to give a compound of the formula (IV) 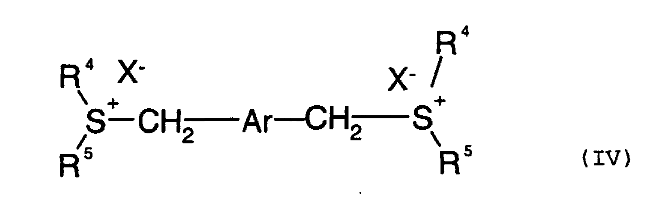 in which R⁴, R⁵, X and Ar are as defined above,
b) reaction of the compound of the formula (IV) with from 0.85 to 1.1 equivalents of a mercaptan of the formula (V)
R³-SH (V)
in which
R³ is unbranched alkyl having 1 to 20 carbon atoms, branched alkyl having 3 to 20 carbon atoms, cyclic alkyl or C₁-C₄-alkyl-substituted cyclic alkyl, phenyl or benzyl, which may be substituted or unsubstituted and/or contain heteroatoms,
to give a compound of the formula (VI) 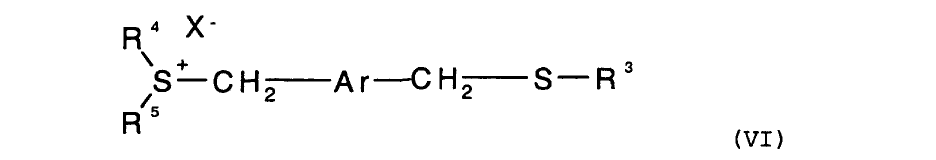
c) warming of the compound of the formula (VI) in a liquid and formation of the compound of the formula (VII) 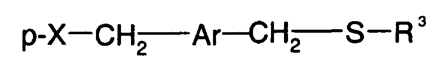 by elimination of an organic sulfide of the formula (III), where the compound of the formula (VII) is dissolved in the above liquid,
d) oxidation of the compound of the formula (VII) using an oxidant, to give the compound of the formula (I).

2. The process as claimed in claim 1, wherein the radical X is a leaving group halogen, -O-tosylate, -O-mesylate or -O-trifluoroacetate.

3. The process as claimed in claim 1 or 2, wherein Ar is the structural unit 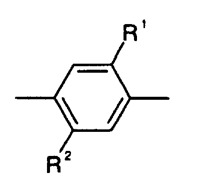 in which
R¹ and R² are identical or different and are hydrogen, an unbranched alkyl or alkoxy radical having 1 to 20 carbon atoms, a branched alkyl or alkoxy radical having 3 to 20 carbon atoms, phenyl or benzyl, where the abovementioned radicals may be unsubstituted or substituted by halogen, cyano, nitro, or an ester having 1 to 20 carbon atoms.

4. The process as claimed in one of claims 1 to 3, wherein Ar is the structural unit 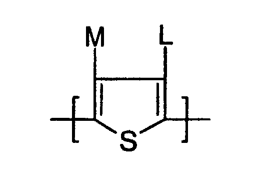 in which
M and L are identical or different and are hydrogen, an unbranched alkyl or alkoxy radical having 1 to 16 carbon atoms, a branched alkyl or alkoxy radical having 3 to 16 carbon atoms, phenyl or benzyl, where the abovementioned radicals may be unsubstituted or substituted by halogen, cyano, nitro, or an ester having 1 to 16 carbon atoms, or M and L together are a bridge having at least 4 bridge members, which may also contain one or more heteroatoms.

5. The process as claimed in claim 3, wherein R¹ and R², independently of one another, are an unbranched alkoxy radical having 1 to 20 carbon atoms, a branched alkoxy radical having 3 to 20 carbon atoms, phenyl or benzyl, where the abovementioned radicals may be unsubstituted or substituted by halogen, cyano, nitro, or an ester having 1 to 20 carbon atoms.

6. The process as claimed in claim 5, wherein R¹ and R², independently of one another, are an unbranched alkoxy radical having 1 to 10 carbon atoms, a branched alkoxy radical having 3 to 20 carbon atoms, or phenyl, which may be substituted by one or more branched or unbranched alkyl or alkoxy groups having up to 20 carbon atoms.

7. The process as claimed in one of claims 1 to 6, wherein R³ is n-, i-, s- or t-butyl, i-pentyl, octyl, 3,6,9-trioxadecyl, 2-hydroxyethyl or 2-chloroethyl.

8. The process as claimed in one of claims 1 to 7, wherein the organic sulfide of the formula (III) is dimethyl sulfide, diethyl sulfide, 2-ethylthioethanol, thiobisethanol, or a cyclic sulfide.

9. The process as claimed in one of claims 1 to 8, wherein the reaction in measure (a) is carried out in methanol, water, ethanol, acetone, dioxane, tetrahydropyran, tetrahydrofuran, acetonitrile or mixtures thereof.

10. The process as claimed in one of claims 1 to 9, wherein the reaction in measure (a) is carried out at temperatures between room temperature (20°C) and the boiling point of the mixture (100°C).

11. The process as claimed in one of claims 1 to 10, wherein the reaction in measure (b) is carried out in the presence of a base.

12. The process as claimed in one of claims 1 to 11, wherein from 0.95 to 1.05 equivalents of mercaptan of the formula (V) are employed per mole of the compound of the formula (IV).

13. The process as claimed in one of claims 1 to 12, wherein the reaction in measure (b) is carried out at between 0°C and 40°C.

14. The process as claimed in one of claims 1 to 13, wherein the reaction in measure (b) is carried out in a polar, protic solvent.

15. A compound of the formula (VI) 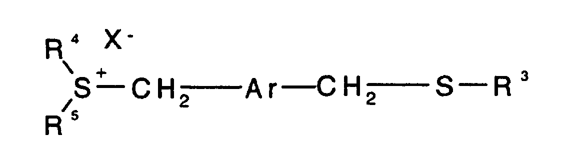 in which
Ar is an aromatic ring system having 4 to 20 carbon atoms, which may, if desired, be monosubstituted or polysubstituted by C₁-C₂₀-alkyl, C₃-C₂₀-alkoxy, C₃-C₂₀-branched alkyl, phenyl or benzyl radicals and which may contain up to 4 heteroatoms from the group consisting of oxygen, sulfur and nitrogen in the aromatic ring system,
X is a leaving group, and
R³ is unbranched alkyl having 1 to 20 carbon atoms, branched alkyl having 3 to 20 carbon atoms, cyclic alkyl, or C₁-C₄-alkyl-substituted cyclic alkyl, phenyl or benzyl, which may be substituted or unsubstituted and/or contain heteroatoms,
R⁴ and R⁵ are identical or different and are unbranched alkyl having 1 to 20 carbon atoms, branched alkyl having 3 to 20 carbon atoms, cyclic alkyl having 3 to 10 carbon atoms, or C₁-C₄-alkyl-substituted cyclic alkyl, or R⁴ and R⁵ together form a ring, which may also contain one or more heteroatoms selected from the group consisting of oxygen, sulfur and nitrogen.

16. A compound as claimed in claim 15, wherein Ar is the structural unit 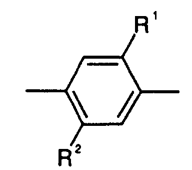 in which
R¹ and R² are identical or different and are hydrogen, an unbranched alkyl or alkoxy radical having 1 to 20 carbon atoms, a branched alkyl or alkoxy radical having 3 to 20 carbon atoms, phenyl or benzyl, where the abovementioned radicals may be unsubstituted or substituted by halogen, cyano, nitro, or an ester having 1 to 20 carbon atoms.

17. A compound as claimed in claim 15, wherein Ar is the structural unit 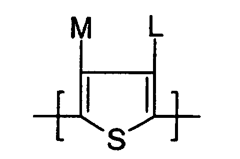 in which
M and L are identical or different and are hydrogen, an unbranched alkyl or alkoxy radical having 1 to 16 carbon atoms, a branched alkyl or alkoxy radical having 3 to 16 carbon atoms, phenyl or benzyl, where the abovementioned radicals may be unsubstituted or substituted by halogen, cyano, nitro, or an ester having 1 to 16 carbon atoms, or M and L together are a bridge having at least 4 bridge members, which may also contain one or more heteroatoms.

## Revendications

1. Procédé pour la préparation de composés de formule (I) 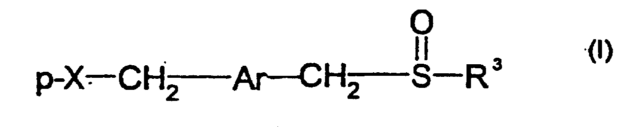 où
Ar représente un système cyclique aromatique présentant 4 à 20 atomes de carbone, qui peut éventuellement être substitué une ou plusieurs fois par des restes alkyle en C₁-C₂₀, alkoxy en C₃-C₂₀, alkyle en C₃-C₂₀ ramifié, phényle ou benzyle, et peut éventuellement contenir dans le système cyclique aromatique jusqu'à 4 hétéroatomes pris parmi les atomes d'oxygène, de soufre ou d'azote,
X est un groupe partant, et
R³ représente un groupe alkyle linéaire présentant 1 à 20 atomes de carbone, un groupe alkyle ramifié présentant 3 à 20 atomes de carbone, un groupe alkyle cyclique, ou un groupe alkyle cyclique substitué par un substituant alkyle en C₁-C₄, phényle ou benzyle, qui peuvent éventuellement être substitués, et/ou contenir des hétéroatomes,
comprenant les mesures
a) réaction d'un composé de formule (II)
p-X-CH₂-Ar-CH₂-X (II)
où Ar et X possèdent les significations données à la formule (I), sur un sulfure organique de formule (III)
R⁴-S-R⁵ (III)
où
R⁴, R⁵ représentent, identiques ou différents, un groupe alkyle linéaire présentant 1 à 20 atomes de carbone, un groupe alkyle ramifié présentant 3 à 20 atomes de carbone, un groupe alkyle cyclique présentant 3 à 10 atomes de carbone, un groupe alkyle cyclique substitué par un substituant alkyle en C₁-C₄, ou R⁴ et R⁵ forment conjointement un cycle qui peut aussi contenir un ou plusieurs hétéroatomes pris parmi les atomes d'oxygène, de soufre et d'azote,
pour obtenir un composé de formule (IV) 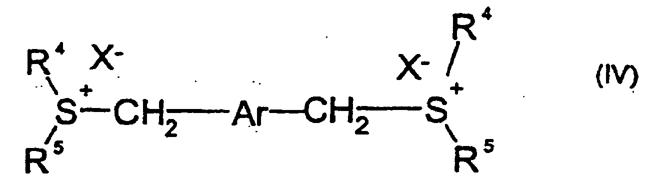 où R⁴, R⁵, X et Ar possèdent la signification donnée auparavant,
b) réaction du composé de formule (IV) sur 0,85 à 1,1 équivalent d'un mercaptan de formule (V)
R³-SH (V)
où
R³ représente un groupe alkyle linéaire présentant 1 à 20 atomes de carbone, un groupe alkyle ramifié présentant 3 à 20 atomes de carbone, un groupe alkyle cyclique ou un groupe alkyle cyclique substitué par un substituant alkyle en C₁-C₄, phényle ou benzyle, qui peuvent éventuellement être substitués, et/ou contenir des hétéroatomes,
pour obtenir un composé de formule (VI) 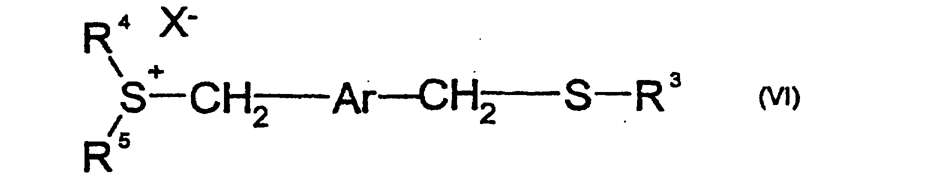
c) chauffage du composé de formule (VI) dans un liquide et formation du composé de formule (VII) 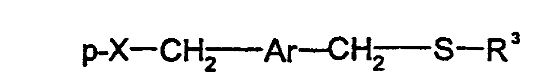 par dissociation d'un sulfure organique de formule (III), le composé de formule (VII) se présente dissous dans le liquide précédent,
d) oxydation des composés de formule (VII) par un agent oxydant pour obtenir le composé de formule (I).

2. Procédé selon la revendication 1, **caractérisé en ce que** le reste X représente un groupe partant halogène, -O-tosylate, -O-mésylate ou -O-trifluoracétate.

3. Procédé selon la revendication 1 ou 2, **caractérisé en ce que** Ar représente une unité de structure 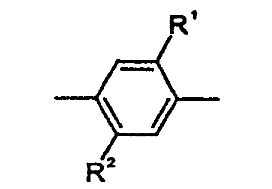 où
R¹, R² représentent, identiques ou différents, un atome d'hydrogène, un groupe alkoxy ou alkyle linéaire présentant 1 à 20 atomes de carbone, un groupe alkoxy ou alkyle ramifié présentant 3 à 20 atomes de carbone, un groupe phényle ou benzyle, les restes précités pouvant éventuellement être substitués, un atome d'halogène, un groupe cyano, nitro ou un ester présentant 1 à 20 atomes de carbone.

4. Procédé selon l'une des revendications 1 à 3, **caractérisé en ce que** Ar représente une unité de structure 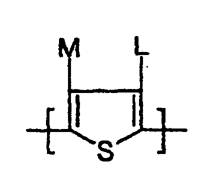 où
M, L sont identiques ou différents et représentent un atome d'hydrogène, un groupe alkoxy ou alkyle linéaire présentant 1 à 16 atomes de carbone, un groupe alkoxy ou alkyle ramifié présentant 3 à 16 atomes de carbone, un groupe phényle ou benzyle, les restes précités pouvant éventuellement être substitués, un atome d'halogène, un groupe cyano, nitro ou un ester présentant 1 à 16 atomes de carbone, ou M et L représentent conjointement un pont comprenant au moins 4 termes de pontage, qui peuvent également contenir un ou plusieurs hétéroatomes.

5. Procédé selon la revendication 3, **caractérisé en ce que** R¹ et R² représentent indépendamment l'un de l'autre les restes un reste alkoxy linéaire présentant 1 à 20 atomes de carbone, un reste alkoxy ramifié présentant 3 à 20 atomes de carbone, un groupe phényle ou benzyle, les restes précités pouvant éventuellement être substitués, un atome d'halogène, un groupe cyano, nitro ou un ester présentant 1 à 20 atomes de carbone.

6. Procédé selon la revendication 5, **caractérisé en ce que** R¹ et R² représentent, indépendamment l'un de l'autre, les restes un reste alkoxy linéaire présentant 1 à 10 atomes de carbone, un reste alkoxy ramifié présentant 3 à 20 atomes de carbone un groupe phényle, qui peut être substitué par un ou plusieurs groupes alkyle ou alkoxy ramifié ou linéaire présentant jusqu'à 20 atomes de carbone.

7. Procédé selon l'une des revendications 1 à 6, **caractérisé en ce que** R³ représente un groupe n-, i-, s-, t-butyle, i-pentyle, octyle, 3,6,9-trioxadécyle, 2-hydroxyéthyle ou 2-chloréthyle.

8. Procédé selon l'une des revendications 1 à 7, **caractérisé en ce qu'**on utilise en tant que sulfure organique de formule (III) un sulfure de diméthyle, un sulfure de diéthyle, le 2-éthylthioéthanol, le thiobiséthanol ou un sulfure cyclique.

9. Procédé selon l'une des revendications 1 à 8, **caractérisé en ce qu'**on met en oeuvre la réaction selon la mesure (a) dans du méthanol, de l'eau, de l'éthanol, de l'acétone, du dioxane, du tétrahydropyrane, du tétrahydrofuranne, de l'acétonitrile ou leurs mélanges.

10. Procédé selon l'une des revendications 1 à 9, **caractérisé en ce qu'**on met en oeuvre la réaction à une température comprise entre la température ambiante (20°C) et la température d'ébullition (100°C) du mélange.

11. Procédé selon l'une des revendications 1 à 10, **caractérisé en ce qu'**on met en oeuvre la réaction selon l'étape (b) en présence d'une base.

12. Procédé selon l'une des revendications 1 à 11, **caractérisé en ce qu'**on utilise par mole du composé de formule (IV) de 0,95 à 1,05 équivalents de mercaptan de formule (V).

13. Procédé selon l'une des revendications 1 à 12, **caractérisé en ce qu'**on met en oeuvre la réaction selon la mesure (b) à une température comprise entre 0°C et 40°C.

14. Procédé selon l'une des revendications 1 à 13, **caractérisé en ce qu'**on met en oeuvre la réaction selon la mesure (b) dans un solvant polaire protique.

15. Composés de formule (VI) 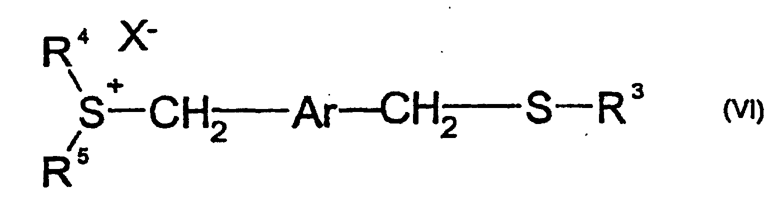 où
Ar représente un système cyclique aromatique présentant 4 à 20 atomes de carbone, qui peut éventuellement être substitué une ou plusieurs fois par des restes alkyle en C₁-C₂₀, alkoxy en C₃-C₂₀, alkyle en C₃-C₂₀ ramifié, phényle ou benzyle, et peut éventuellement contenir dans le système cyclique aromatique jusqu'à 4 hétéroatomes pris parmi les atomes d'oxygène, de soufre ou d'azote,
X est un groupe partant, et
R³ représente un groupe alkyle linéaire présentant 1 à 20 atomes de carbone, un groupe alkyle ramifié présentant 3 à 20 atomes de carbone, un groupe alkyle cyclique, ou un groupe alkyle cyclique substitué par un substituant alkyle en C₁-C₄, phényle ou benzyle, qui peuvent éventuellement être substitués et/ou contenir des hétéroatomes,
R⁴, R⁵ représentent, identiques ou différents, un groupe alkyle linéaire présentant 1 à 20 atomes de carbone, un groupe alkyle ramifié présentant 3 à 20 atomes de carbone, un groupe alkyle cyclique présentant 3 à 10 atomes de carbone, un groupe alkyle cyclique substitué par un substituant alkyle en C₁-C₄, ou R⁴ et R⁵ forment conjointement un cycle qui peut aussi contenir un ou plusieurs hétéroatomes pris parmi les atomes d'oxygène, de soufre et d'azote.

16. Composés selon la revendication 15, **caractérisés en ce que** Ar représente une unité de structure 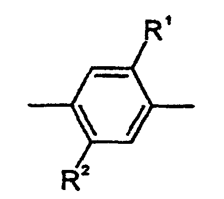
R¹, R² sont identiques ou différents et représentent un atome d'hydrogène, un reste alkoxy ou alkyle linéaire présentant 1 à 20 atomes de carbone, un reste alkoxy ou alkyle ramifié présentant 3 à 20 atomes de carbone, un groupe phényle ou benzyle, les restes précités pouvant éventuellement être substitués, un atome d'halogène, un groupe cyano, nitro ou un ester présentant 1 à 20 atomes de carbone.

17. Composés selon la revendication 15, **caractérisés en ce que** Ar représente unité de structure 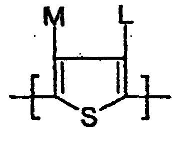 où
M, L sont identiques ou différents et représentent un atome d'hydrogène, un groupe alkoxy ou alkyle linéaire présentant 1 à 16 atomes de carbone, un groupe alkoxy ou alkyle ramifié présentant 3 à 16 atomes de carbone, un groupe phényle ou benzyle, les restes précités pouvant éventuellement être substitués, un atome d'halogène, un groupe cyano, nitro ou un ester présentant 1 à 16 atomes de carbone, ou M et L représentent conjointement un pont comprenant au moins 4 termes de pontage, qui peuvent également contenir un ou plusieurs hétéroatomes.
